(19) Europäisches Patentamt

European Patent Office

Office européen des brevets

(11) Veröffentlichungsnummer: **0 242 708 B1**

# (12) EUROPÄISCHE PATENTSCHRIFT

(45) Veröffentlichungstag der Patentschrift: **19.11.92**

(51) Int. Cl.⁵: **A61F 2/44**

(21) Anmeldenummer: **87105186.8**

(22) Anmeldetag: **08.04.87**

(54) **Pedikelschraube.**

(30) Priorität: **25.04.86 DE 3614101**

(43) Veröffentlichungstag der Anmeldung:
**28.10.87 Patentblatt 87/44**

(45) Bekanntmachung des Hinweises auf die
Patenterteilung:
**19.11.92 Patentblatt 92/47**

(84) Benannte Vertragsstaaten:
**AT BE CH DE ES FR GB IT LI LU NL SE**

(56) Entgegenhaltungen:
**EP-A- 0 242 842**
**WO-A-80/01137**
**FR-A- 2 405 063**
**FR-A- 2 499 400**
**US-A- 4 611 582**

**PROCEEDINGS OF THE ELEVENTH ANNUAL
NORTHEAST BIOENGINEERING CONFEREN-
CE,Worcester, Massachusetts, 14.-15. März
1985, Seiten 313-317, IEEE; M.H. KRAG etal.:
"Vermont spinal fixator for posterior thoracolumbar or lumbosacral spine stabilization:
initial mechanical testing and implantation"**

(73) Patentinhaber: **HARMS, Jürgen, Prof. Dr.**
**Am Rüppurrer Schloss 5**
**W-7500 Karlsruhe(DE)**

Patentinhaber: **Biedermann, Lutz**
**Am Schäfersteig 8**
**W-7730 VS-Villingen(DE)**

(72) Erfinder: **HARMS, Jürgen, Prof. Dr.**
**Am Rüppurrer Schloss 5**
**W-7500 Karlsruhe(DE)**
Erfinder: **Biedermann, Lutz**
**Am Schäfersteig 8**
**W-7730 VS-Villingen(DE)**

(74) Vertreter: **Prüfer, Lutz H., Dipl.-Phys.**
**Harthauser Strasse 25d**
**W-8000 München 90(DE)**

Rank Xerox (UK) Business Services

# Beschreibung

Die Erfindung betrifft eine Pedikelschraube mit einem Gewindeschaftteil und einem kopfseitig vorgesehenen Aufnahmeteil für eine Stange. Derartige Schrauben werden zur mono- oder multisegmentalen Stabilisierung der Wirbelsäule verwendet.

Eine derartige Pedikelschraube ist aus der DE-AS 26 49 042 bekannt. Die Schraube weist einen Gewindeteil und einen starr damit am kopfseitigen Ende vorgesehenen Aufnahmeteil auf. Es werden mehrere Paare solcher Schrauben jeweils in einem Abstand voneinander beidseitig von der Wirbelsäule in die Wirbelkörper eingeschraubt. Die jeweiligen Aufnahmeteile weisen Aufnahmeschlitze auf. Durch diese Aufnahmeschlitze der rechten bzw. linken Gruppe der Schrauben wird jeweils eine Gewindestange geführt. Mit Hilfe von Fixierungsschrauben wird die Stange dann am jeweiligen Aufnahmeteil fixiert. Ein Nachteil dieser Lösung besteht darin, daß es sehr schwierig ist, die Schrauben einerseits fest in die Wirbelkörper einzuschrauben und andererseits die Schrauben in zwei Ebenen gerade so zu stellen, daß die Achsen der Aufnahmeschlitze in den übereinander befindlichen Aufnahmeteilen so ausgerichtet sind, daß die Gewindestange ohne Verspannung der Schrauben durch die Aufnahmeschlitze hindurchführbar ist. Schon der Versuch erfordert sehr viel Zeit, was bei einer Operation an der Wirbelsäule ein großer Nachteil ist. Darüberhinaus läßt sich eine so genaue Ausrichtung fast nicht erreichen. Das Ergebnis ist, daß erhebliche Scherkräfte auf die Gewindestangen ausgeübt werden, was dazu führt, daß in der späteren Benutzung nach Abschluß der Operation die Stangen sogar abbrechen könnten bzw. nicht die volle Stabilisierung ermöglichen.

Aus der FR-A-2 499 400 ist eine Pedikelschraube bekannt, bei der das Gewindeschaftteil und das Aufnahmeteil über eine aus zwei Segmenten bestehende Kugel schwenkbar verbunden werden. Diese Anordnung ist konstruktiv sehr voluminös und kann daher nur für eine externe Fixation von Röhrenknochen, nicht aber für die interne Stabilisierung von Wirbelsäulensegmenten eingesetzt werden.

Aufgabe der Erfindung ist es, eine Pedikelschraube der eingangs beschriebenen Art zu schaffen, die das Einsetzen erleichtert und gleichzeitig auch die Gefahren beim späteren Benutzen vermindert bzw. ausschließt.

Diese Aufgabe wird durch eine Pedikelschraube nach Anspruch 1 gelöst.

Durch diese Lösung wird erreicht, daß die Schraube zunächst ohne Rücksicht auf die Stellung des Aufnahmeteiles eingeschraubt werden kann und das Aufnahmeteil anschließend leicht in eine für die Aufnahme der Gewindestange erforderliche Stellung bringbar ist. Dadurch wird die Operationszeit wesentlich verkürzt, und die auf die Gewindestange wirkenden Kräfte werden wesentlich verringert.

Weitere Merkmale und Zweckmäßigkeiten der Erfindung ergeben sich aus der Beschreibung von Ausführungsbeispielen anhand der Figuren. Von den Figuren zeigen:

Fig. 1      eine erste Ausführungsform einer Pedikelschraube im Schnitt, in vergrößerter Darstellung;

Fig. 2      einen Teil aus Fig. 1 in vergrößertem Maßstab;

Fig. 3      eine Draufsicht auf den in Fig. 2 gezeigten Teil;

Fig. 4      ein Teil aus Fig. 1 in geändertem Maßstab;

Fig. 5      das in Fig. 4 gezeigte Teil in einer um 90° gedrehten Darstellung im Schnitt;

Fig. 6      das in Fig. 4 gezeigte Teil von der Rückseite;

Fig. 7      das in Fig. 5 gezeigte Teil von oben; und

Fig. 8      einen Haltering aus der in Fig. 1 gezeigten Darstellung in vergrößertem Maßstab.

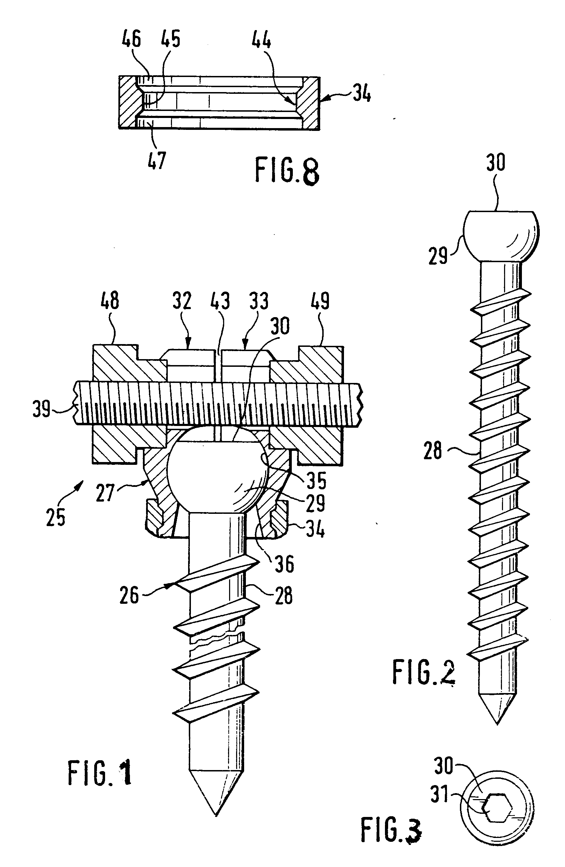

Die in den Figuren 1 bis 8 dargestellte Pedikelschraube 25 weist einen Gewindeschaftteil 26 und ein Aufnahmeteil 27 auf.

Der Gewindeschaftteil 26 umfaßt einen Gewindeteil 28 zum Einschrauben in die Wirbelkörper. Am kopfseitigen Ende des Gewindeteiles ist ein kugelsegmentförmiger Kopf 29 vorgesehen. Dieser weist auf seiner dem Gewindeteil abgewandten Seite eine sich senkrecht zur Achse des Gewindeteiles 28 erstreckende ebene Fläche 30 auf, die dadurch gebildet ist, daß ein Abschnitt der Kugel abgeschnitten ist. Der abgeschnittene Bereich entspricht in etwa einem halben Radius des Kugelkörpers.

Wie aus Figur 3 ersichtlich ist, ist koaxial zu dem Gewindeteil 28 eine Sechskantbohrung 31 vorgesehen. In diese kann mit einem Imbusschlüssel zum Einschrauben des Gewindeschaftteiles 26 in den Wirbelkörper eingegriffen werden. Anstelle der Sechskantbohrung können natürlich auch andere Formen zum Eingreifen mittels eines Schraubendrehers vorgesehen sein.

Das Aufnahmeteil 27 umfaßt zwei Kopfhälften 32, 33 sowie einen diese zusammenhaltenden Haltering 34.

Jede Kopfhälfte weist auf ihrer der anderen Kopfhälfte Zugewandten Innenseite einen kugelsegmentförmigen Abschnitt 35 auf. Der Innenradius der Kugel entspricht dem Außenradius des Kopfes 29. An den kugelsegmentförmigen Abschnitt schließt sich ein Halsabschnitt 36 an. Dieser hat die Form eines Segmentes eines Kegelabschnittes und ist von dem kugelsegmentförmigen Abschnitt ausgehend nach außen divergent ausgebildet. Die Achse des Halsabschnittes geht durch den Mittelpunkt des kugelsegmentförmigen Abschnittes 35. Auf der dem Halsabschnitt gegenüberliegenden Seite des kugelsegmentförmigen Abschnittes 35 erstreckt sich senkrecht zu der Symmetrieachse von Halsabschnitt und kugelsegmentförmigem Abschnitt ein Aufnahmeschlitz 37, an dessen der anderen Kopfhälfte abgewandten Außenseite eine Senkung 38 vorgesehen ist. Die Breite des Aufnahmeschlitzes ist so gewählt, daß eine aufzunehmende Gewindestange 39 lose durch diesen hindurchführbar ist, wie dies aus Figur 1 ersichtlich ist.

Im Bereich des Halsabschnittes 36 weist die Kopfhälfte eine sich senkrecht zur Symmetrieachse von Halsabschnitt und kugelsegmentförmigem Abschnitt erstreckende nutenförmige Ausnehmung 40 auf.

Die beiden Kopfhälften 32, 33 sind identisch ausgebildet. Ihre Abmessungen sind so gewählt, daß der Mittelpunkt 41 des kugelsegmentförmigen Abschnittes 35 jeweils einen Bruchteil eines Millimeters außerhalb der der jeweiligen zweiten Kopfhälfte gegenüberliegenden Trennebene 42 liegt, so daß bei Zusammenfügen der beiden Kopfhälften 32, 33 parallel zueinander um den Kopf ein Spalt 43 zwischen den beiden Hälften entsteht.

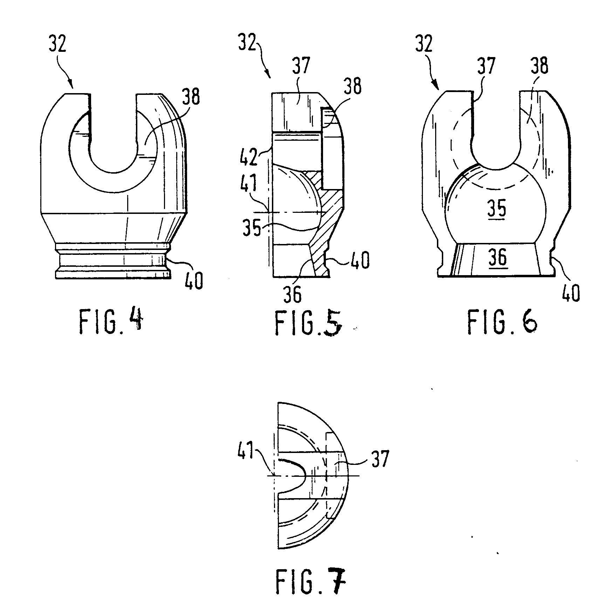

Der Haltering 34 weist eine innere Oberfläche 44 auf, die praktisch die Negativform der Außenfläche des Halsabschnittes 36 ist. Insbesondere weist die innere Oberfläche 44 einen in die nutenförmige Ausnehmung 40 hineinpassenden hervorstehenden Wulst 45 mit jeweils daran angrenzenden seitlichen Randbereichen 46, 47 auf. Der Innendurchmesser des Halteringes 34, also insbesondere des Wulstes 45 und der Randbereiche 46, 47 ist so gewählt, daß der Ring 34 eine solche Spannung auf die beiden Kopfhälften 32, 33 ausübt, wenn diese in der in Figur 4 gezeigten Weise zunächst noch ohne eingesetzte Gewindestange 39 auf den Kopf 29 aufgesetzt sind, daß sie innerhalb eines durch die Konizität des Halsabschnittes 36 bestimmten Winkels frei um die Längsachse des Gewindeschaftteiles 26 schwenkbar sind und für das Einsetzen der Gewindestange 39 ausreichend fest mit dem Kopf und somit mit dem Gewindeschaftteil 26 so verbunden sind, daß die beiden Kopfhälften in dem von dem Ring umfaßten Bereich weiter zusammen sind als in dem gegenüberliegenden, den Aufnähmeschlitz aufweisenden Bereich.

Wie sich insbesondere aus den Figuren 1 und 6 ergibt, ist die Tiefe des Aufnahmeschlitzes 37 so gewählt, daß dieser in dem in Figur 1 gezeigten zusammengesetzten Zustand in einem Abstand über der Fläche 30 endet, damit die freie Beweglichkeit des Aufnahmeteiles 27 um die Längsachse des Gewindeschaftteiles 26 nicht eingeschränkt wird.

Zum Einsetzen der Pedikelschraube wird zunächst die Pedikelschraube in die jeweiligen Wirbelkörper dadurch eingeschraubt, daß mit einem Schlüssel durch die Aufnahmeschlitze hindurch in die Sechskantöffnung zum Schrauben eingegriffen wird. Nach dem Einschrauben werden die Aufnahmeteile 27 mit den beiden durch den Haltering 34 zusammengehaltenen Kopfhälften 32, 33 so in ihrer Richtung ausgerichtet, daß die Gewindestangen 39 ohne Einwirkung von Scherkräften in die Aufnahmeschlitze 37 einsetzbar sind. Die Gewindestangen werden dann jeweils mit in den Senkungen 38 sitzenden Fixierschrauben 48, 49 fixiert. Die Fixierschrauben können dabei je nach Wunsch entweder so fest angezogen werden, daß das Aufnahmeteil 27 sich nicht mehr um den Kopf 29 herum verschwenken läßt und somit eine im wesentlichen starre Verbindung zwischen Aufnähmeteil 27 und Gewindeschaftteil 26 entsteht, oder die Schrauben werden nur so weit angezogen, daß noch eine gewünschte gedämpfte Bewegung zwischen den beiden Teilen möglich ist.

Bei der in Figur 1 gezeigten Ausführungsform ist der Haltering 34 auf seiner dem Gewindeteil 28 zugewandten Seite um einen Radius abgerundet, dessen Mittelpunkt auf der dem Gewindeteil 28 entgegengesetzten Seite liegt. Durch diese Abrundung wird erreicht, daß die dem jeweiligen Wirbelkörper zugewandten Flächen keinerlei Verletzungen hervorrufen können.

Bei einer Ausführungsform sind die Oberflächen der Ausnehmungen und/oder des kugelsegmentförmigen Kopfes 29 poliert oder mit einem Gleitmittel wie Teflon beschichtet, so daß auch bei relativ festem Anschrauben eine geringe gedämpfte Bewegung des Aufnahmeteils möglich ist. Nach einer anderen Ausführungsform können die Oberflächen rauh ausgebildet sein, so daß sich die Kugel im eingesetzten Zustand mit festangezogener Mutter quasi festfrißt und eine im wesentlichen starre Verbindung zwischen dem Aufnahmeteil und dem Gewindeschaftteil entsteht.

Mit der oben beschriebenen Pedikelschraube wird eine um die Symmetrieachse unbegrenzte freie Bewegung und in horizontaler und vertikaler Richtung eine voreinstellbare Bewegung, die vorzugsweise bei einem Winkel von 10 bis 30° liegt, erreicht. Zusätzlich wird erreicht, daß die Baulänge der Pedikelschraube besonders kurz gehalten werden kann.

**Patentansprüche**

1. Pedikelschraube (25) zur Stabilisierung von Wirbelsäulensegmenten mit einem Gewindeschaftteil (26) und einem kopfseitig vorgesehenen und mit dem Gewindeschaftteil (26) gelenkig verbundenen Aufnahmeteil (27) für eine Stange (39),

wobei das Gewindeschaftteil (26) und das Aufnahmeteil (27) über ein Kugelgelenk miteinander verbunden sind,

dadurch gekennzeichnet, daß das Aufnahmeteil (27) aus zwei jeweils einen innenliegenden kugelsegmentförmigen Abschnitt (35) aufweisenden Kopfhälften (32, 33) besteht und daß das Gewindeschaftteil (26) an seinem dem Gewindeteil (28) abgewandten Ende als kugelsegmentförmiger Kopf (29) ausgebildet ist, auf dem die Kopfhälften (32, 33) mit ihren innenliegenden kugelsegmentförmigen Abschnitten (35) aufliegen.

2. Pedikelschraube nach Anspruch 1,
dadurch gekennzeichnet, daß die Mittelpunkte (41) der Abschnitte (35) so gelegt sind, daß zwischen den Kopfhälften (32, 33) ein Spalt (43) entsteht, wenn diese am Kopf (29) anliegen.

3. Pedikelschraube nach Anspruch 1,
dadurch gekennzeichnet, daß der Kopf (29) eine durch das Aufnahmeteil (27) hindurch zugängliche Einrichtung (31) zum Ineingriffbringen mit einem Schraubendreher aufweist.

4. Pedikelschraube nach Anspruch 1,
dadurch gekennzeichnet, daß die beiden Kopfhälften (32, 33) an ihrem unteren Ende durch einen Ring (34) zusammengehalten werden.

5. Pedikelschraube nach Anspruch 4,
dadurch gekennzeichnet, daß die Kopfhälften (32, 33) an ihrem dem Gewindeschaftteil (26) zugewandten Rand eine nutenförmige Vertiefung aufweisen und der Ring (35) auf seiner Innenseite einen in diese Vertiefung eingreifenden Wulst (45) aufweist.

6. Pedikelschraube nach Anspruch 1,
dadurch gekennzeichnet, daß die Stange (39) als Gewindestange ausgebildet ist, welche wenigstens eine Mutter (48, 49) zum Verbinden der Stange mit dem Aufnahmeteil (27) aufweist.

## Claims

1. A pedicle screw (25) for stabilizing vertebral column segments, with a threaded shaft portion (26) and a holding portion (27) for a bar (39), provided on the side of the head and articulated to the threaded shaft portion (26), wherein the threaded shaft portion (26) and the holding portion (27) are interconnected by a ball joint,

characterized in that the holding portion (27) consists of two head halves (32, 33) respectively having one internal spherical segment section (35), and that at its end remote from the threaded portion (28), the threaded shaft portion (26) is formed as a spherical segment head (29) whereon the head halves (32, 33) bear with their internal spherical segment sections (35).

2. A pedicle screw according to claim 1,
characterized in that the centres (41) of the sections (35) are disposed in such a way that a gap (43) is formed between the head halves (32, 33) when the latter bear on the head (29).

3. A pedicle screw according to claim 1,
characterized in that the head (29) has a device (31) accessible through the holding portion (27) for producing the engagement by means of a screw driver.

4. A pedicle screw according to claim 1,
characterized in that the two head halves (32, 33) are held together at their bottom end by a ring (34).

5. A pedicle screw according to claim 4,
characterized in that at their edge facing the threaded shaft portion (26), the head halves (32, 33) have a groove-shaped recess, and that the ring (34) has on its inner side a bulge (45) engaging in this recess.

6. A pedicle screw according to claim 1,
characterized in that the bar (39) is designed as a threaded bar having at least one nut (48, 49) for connecting the bar to the holding portion (27).

## Revendications

1. Vis pédiculaire (25) destinée à la stabilisation de segments de colonne vertébrale avec un élément de tige filetée (26) et un élément de logement (27) prévu côté tête, et qui est relié de façon articulée à l'élément de tige filetée (26) pour recevoir une tige (39),

l'élément de tige filetée (26) étant relié à l'élément de logement (27) par une articulation sphérique,

caractérisée en ce que l'élément de logement (27) est constitué de deux moitiés de tête (32, 33) comportant chacune une surface intérieure (35) ayant la forme d'un segment sphérique, et en ce que l'extrémité opposée à la partie filetée (28) de l'élément de tige filetée (26) est réalisée sous forme d'une tête (29) ayant la forme d'un segment sphérique, sur laquelle s'appuient les surfaces intérieure (35) des deux moitiés de tête (32, 33) ayant la forme de segments sphériques.

2. Vis pédiculaire selon la revendication 1, caractérisée en ce que les centres (41) des surfaces (35) sont positionnés de sorte qu'il subsiste une fente (43) entre les deux moitiés de tête (32, 33) lorsque celles-ci adhèrent à la tête (29).

3. Vis pédiculaire selon la revendication 1, caractérisée en ce que la tête (29) comporte un dispositif (31) accessible par l'élément de logement (27) pour une intervention au moyen d'un tournevis.

4. Vis pédiculaire selon la revendication 1, caractérisée en ce que les deux moitiés de tête (32, 33) sont maintenues ensemble par une bague (34) en leurs parties inférieures.

5. Vis pédiculaire selon la revendication 4, caractérisée en ce que le bord des deux moitiés de tête (32, 33) comporte un creux sous forme d'encoche du côté de l'élément de tige filetée (26) et en ce que la partie intérieure de la bague (35) comporte un bourrelet (45) s'insérant dans ce creux.

6. Vis pédiculaire selon la revendication 1, caractérisée en ce que la tige (39) est réalisée sous forme d'une tige filetée comportant au moins un écrou (48, 49) pour relier la tige à l'élément de logement (27).

FIG.8

FIG.1

FIG.2

FIG.3

FIG.4

FIG.5

FIG.6

FIG.7